(19) Europäisches Patentamt
European Patent Office
Office européen des brevets

(11) Veröffentlichungsnummer : **0 024 047**
**B1**

(12) # EUROPÄISCHE PATENTSCHRIFT

(45) Veröffentlichungstag der Patentschrift : **09.03.83**

(51) Int. Cl.$^3$ : **C 07 C127/15**, C 08 G 18/78

(21) Anmeldenummer : **80104696.2**

(22) Anmeldetag : **09.08.80**

(54) **Isocyanatomethylgruppenhaltige Harnstoffderivate des 3(4), 8(9)-Diisocyanatomethyltricyclo (5.2.1.0 2,6) decans, Verfahren zu Ihrer Herstellung sowie die Verwendung zur Herstellung von Polyurethan-Elastomeren.**

(30) Priorität : **11.08.79 DE 2932646**

(43) Veröffentlichungstag der Anmeldung : **18.02.81 Patentblatt 81/07**

(45) Bekanntmachung des Hinweises auf die Patenterteilung : **09.03.83 Patentblatt 83/10**

(84) Benannte Vertragsstaaten : **BE CH DE FR GB IT LI LU NL SE**

(56) Entgegenhaltungen :
**DE A 1 645 595**
**DE A 2 032 547**
**FR A 2 200 255**

(73) Patentinhaber : **CHEMISCHE WERKE HÜLS AG**
**Postfach 1320**
**D-4370 Marl 1 (DE)**

(72) Erfinder : **Schnurbusch, Horst, Dr.**
**Overwegstrasse 36**
**D-4690 Herne 1 (DE)**
Erfinder : **Gras, Rainer, Dr.**
**An der Ziegelei 91**
**D-4690 Herne 2 (DE)**
Erfinder : **Wolf, Elmar, Dr.**
**Am Böckenbusch 3a**
**D-4690 Herne 2 (DE)**

(74) Vertreter : **Steil, Hanna, Dipl.-Chem.**
**RSP PATENTE - PB 15 Postfach 1320**
**D-4370 Marl 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 024 047 B1

Isocyanatomethylgruppenhaltige Harnstoffderivate des 3(4), 8(9)-Diisocyanatomethyltricyclo [5.2.1.$0^{2,6}$] decans, Verfahren zu ihrer Herstellung sowie die Verwendung zur Herstellung von Polyurethan-Elastomeren

Beschreibung und Beispiele

Die vorliegende Erfindung betrifft isocyanatomethylgruppenhaltige Harnstoffderivate des 3(4), 8(9)-Diisocyanatomethyltricyclo- [5.2.1.$0^{2,6}$] decans, welches im folgenden als TCDI bezeichnet werden soll, sowie ihre Herstellung und Verwendung. Kalthärtende hochelastische Polyurethanmassen haben in den letzten Jahren große Bedeutung erlangt. Dazu haben besonders ihre hervorragenden Eigenschaften, wie ihre gute Abrieb- und Chemikalienbeständigkeit, ihre Zähigkeit, ihre Dauerelastizität, ihre Strapazierfähigkeit und ihre Tieftemperaturbeständigkeit, beigetragen.

Für derartige lösungsmittelfreie Polyurethan-Elastomere läßt sich vorteilhaft ein in der DE-OS 23 41 065 beschriebenes Harnstoff-Derivat des Isophorondiisocyanats einsetzen. Ein Nachteil dieses Harnstoff-Derivats besteht allerdings in der großen Reaktionsträgheit seiner Isocyanatgruppen. Zur Aushärtung dieses Harnstoff-Derivats mit einer entsprechenden Polyolkomponente sind unbedingt mehr oder weniger große Mengen an Katalysatoren notwendig. Diese Katalysatoren beschleunigen aber nicht nur die Reaktion zwischen NCO- und HO- Gruppen, sie beeinträchtigen auch, wenn nur in begrenztem Umfang, die Hydrolysestabilität des ausgehärteten Polyurethansystems.

Aufgabe der vorliegenden Erfindung war es, ein Diisocyanat aufzuzeigen, das die Vorteile des Isophorondiisocyanat-Harnstoff-Derivats besitzt, aber nicht dessen Nachteile.

Gegenstand der Erfindung sind isocyanatomethylgruppenhaltige Harnstoffderivate des 3(4), 8(9)-Diisocyanatomethyltricyclo [5.2.1.$0^{2,6}$] decans (TCDI) der Formel

$$OCN—H_2C—[R—CH_2—NH—CO—NH—CH_2]_n—R—CH_2—NCO,$$

in der R

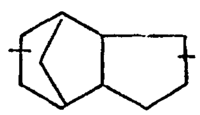

und n = 1-5 ist.

Es wurde weiter gefunden, daß man diese Verbindungen leicht durch Umsetzen des 3(4), 8(9)-Diisocyanatomethyltricyclo [5.2.1.$0^{2,6}$] decans (TCDI)

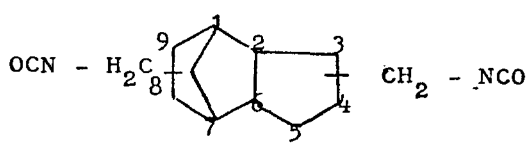

mit unterschüssigen Mengen von Wasser erhalten kann, indem man TCDI mit Wasser im Molverhältnis 12-2,0 : 1, vorzugsweise 6-3 : 1, bei 60-110 °C, vorzugsweise 80-100 °C, gegebenenfalls in Gegenwart eines Katalysators und/oder eines inerten Lösungsmittels umsetzt. Als Umsetzungsprodukte des TCDI entstehen neben entweichendem $CO_2$ Verbindungen der vorstehenden allgemeinen Formel.

Das Reaktionsprodukt kann sowohl nicht umgesetztes TCDI (n = 0) als auch Reaktionsprodukte enthalten, für die n auch bis zu 8 ist. Normalerweise liegt für das Reaktionsprodukt n im Mittel zwischen 1 und 5.

Es ist — wie das Reaktionsprodukt von Isophorondiisocyanat mit Wasser — sowohl in überschüssigem Diisocyanat, als auch in einer Reihe von Polyurethan-Lösungsmitteln löslich. Das erfindungsgemäß partiell mit Wasser umgesetzte TCDI zeichnet sich durch seine hohe Reaktivität, sowie durch seine niedrige Viskosität aus. Es ist überraschend gut zur Herstellung lösungsmittelfreier Polyurethan-Elastomere geeignet, die sich durch gute Farbstabilität und Weiterreißfestigkeit auszeichnen.

Die Herstellung der isocyanatomethylgruppenhaltigen Harnstoffderivate des TCDI der vorstehenden allgemeinen Formel erfolgt in der Weise, daß man zu dem auf 60-110 °C erwärmten TCDI langsam Wasser im Molverhältnis TCDI : Wasser von 12 bis 2,0 : 1 zusetzt. Besonders interessante Produkte erhält man mit Molverhältnissen zwischen 6-3 : 1.

Als besonders zweckmäßig hat es sich erwiesen, wenn das Wasser dampfförmig zugegeben wird. Das erreicht man am einfachsten dadurch, daß ein mit Wasser gesättigter Stickstoffstrom durch das TCDI hindurchgeleitet wird. Nach dieser Verfahrensvariante werden höhermolekulare Harnstoff-Derivate (n > 2) weitgehend unterdrückt.

Als Katalysatoren für den Umsatz des TCDI mit Wasser sind metallorganische Verbindungen geeignet oder Carbonsäuresalze des Sn, Pb, Zn, Fe oder Mn. Beispiele für derartige Katalysatoren sind Di-n-butylzinndilaurat und -diacetat, Zinn(II)-octoat oder Zinkoctoat. Außerdem können tertiäre Amine, wie 1,4-Diazabicyclooctan [2.2.2] allein oder in Mischungen mit den zuvor genannten Verbindungen, als Katalysatoren verwendet werden.

Die erfindungsgemäßen Verbindungen lassen sich mit den verschiedensten hydroxylgruppenhaltigen Verbindungen ohne Schwierigkeiten zu Polyurethan-Elastomeren umsetzen. Im Gegensatz zu dem in der DE-OS 23 41 065 beschriebenen Harnstoffderivat des Isophorondiisocyanats wird bei den TCDI-Derivaten zur Aushärtung bei Raumtemperatur kein Katalysator benötigt.

Folgende hydroxylgruppenhaltigen Verbindungen sind als Umsetzungskomponenten möglich :

Polyesterpolyole, Kondensationsprodukte aus aliphatischen und/oder aromatischen Polycarbonsäuren, wie Adipinsäure, Azelainsäure, Sebacinsäure, Dodecandicarbonsäure, Phthalsäure, Isophthalsäure, Tetrahydrophthalsäure, Hexahydrophthalsäure u. a. ; und Glykolen, wie Äthylenglykol, Diäthylenglykol, Triäthylenglykol, Propandiol-1.3(1.2), Butandiol-1.4(1.3), Pentandiol-1.5, 3-Methylpentandiol-1.5, Hexandiol-1.6, 2.2.4-(2.4.4.)-Trimethylhexandiol-1.6, Neopentylglykol, Hydroxypivalinsäureneopentylglykolester, 3(4),8(9)-Dihydroxymethyl-tricyclo-[5.2.1.$0^{2,6}$]-decan, Dimethylolcyclohexan u. a. ; sowie Polyolen, wie 1.1.1-Trimethyloläthan, 1.1.1-Trimethylolpropan, Glyzerin, Hexantriol-1.2.6 u. a. Polylactonpolyesterpolyole ; Polymerisationsprodukte von Lactonen, wie ε-Caprolacton, ε-Methylcaprolacton, Valerolacton u. a. ; Polyätherpolyole, die durch anionische Polymerisation, Copolymerisation und Blockpolymerisation von Alkylenoxiden, wie Äthylenoxid, Propylenoxid oder Butylenoxid, mit di- oder polyfunktionellen Alkoholen, wie Butandiol-1.4, 1.1.1-Trimethyloläthan, 1.1.1-Trimethylolpropan, Glyzerin, Pentaerythrit und Sorbit bzw. deren Alkalialkoholaten oder mit Aminen, wie Methylamin und Äthylendiamin, als Startkomponente oder durch kationische Polymerisation bzw. Copolymerisation cyclischer Äther, wie Tetrahydrofuran, Äthylenoxid oder Propylenoxid mit sauren Katalysatoren, wie Bortrifluoridätherat oder durch Polykondensation von unter Wasserabspaltung polykondensierbaren Glykolen, wie Hexandiol-1.6, in Gegenwart saurer Verätherungskatalysatoren, wie p-Toluolsulfonsäure, erhalten werden können.

Alle diese Verbindungen können einzeln sowie in Mischungen eingesetzt werden. (Vergleiche auch die Versuchsbeispiele) Isocyanate und hydroxylgruppenhaltige Verbindungen werden in solche Mengen eingesetzt, daß das NCO/OH-Gruppenverhältnis 0,9-1,1 : 1, vorzugsweise 0,95-1,05 : 1, beträgt.

Außer den Reaktionspartnern können gegebenenfalls die in der Polyurethan-Chemie üblichen Hilfs- und Zusatzstoffe verwendet werden :

1. Eigenschaftsverbessernde Füllstoffe
Quarzmehl, Kreide, Schiefermehl, Aluminiumtrioxidhydrat, sowie Gewebe, Fasern, Schnitzel aus Glas, Textil und Gummi.

2. Wasseradsorber,
wie Alkali-Alumo-silikat mit Zeolithstruktur. Wenn es nicht gelingt, das Reaktionsgemisch allein durch Vakuum zu entgasen und zu entfeuchten, so kann der Reaktionsmasse Zeolith zugesetzt werden, um blasenfreie Polyurethan-Elastomere zu erhalten.

3. Reaktive, Verdünner wie Aryloxyalkanole z. B. Verdünner PU (Hersteller Fa. Bayer AG), ein Umsetzungsprodukt aus Phenol und Propylenoxid.

4. Flammhemmer, Weichmacher, Pigmente, Stabilisatoren, Entlüfter, Netzmittel etc.

Die Vermischung der Komponenten kann manuell oder maschinell erfolgen. Die maschinelle Mischung ist insbesondere dann ratsam, wenn eine kontinuierliche Verarbeitung vorgesehen ist.

Die Applikation der erfindungsgemäß erhaltenen Mischungen kann nach verschiedenen bekannten Methoden-, wie Gießen, Spritzen, Streichen, Rakeln und Spachteln-, mit den hierzu gebräuchlichen Werkzeugen und Maschinen erfolgen.

## Beispiele

### A. Herstellung der Polyolkomponenten

1. 292 g Adipinsäure, 104 g Neopentylglykol, 118 g Hexandiol-1.6 und 134 g 1.1.1-Trimethylolpropan wurden zur Veresterung gebracht. Nach Abspaltung von 4 Mol Wasser wurde unter Zusatz von 0,1 Gew.% Di-n- butylzinnoxid (DBTO) die Veresterung bis zu einer Säurezahl $< 2$ mg KOH/g fortgesetzt. Durch Evakuieren auf 13.33 Pa bei 180 °C wurde nach 20-30 Minuten ein Produkt mit einem Wassergehalt von $< 0,1$ Gew.%, einer Hydroxylzahl von 270-285 mg KOH/g und einer Viskosität (bei 25 °C) von ca. 500 Pa · s.

2. 2 685 g Adipinsäure, 944 g Hexandiol-1.6, 1 650 g Triäthylenglykol und 268 g 1.1.1-Trimethylolpropan wurden wie in A 1 unter Katalysator-Zusatz zur Veresterung gebracht. Der so gewonnene Polyester hatte schließlich eine Säurezahl von $< 2$ mg KOH/g, eine Hydroxylzahl von 89-91 mg KOH/g und eine Viskosität (bei 25 °C) von ca. 500 Pa · s.

3. 438 g Adipinsäure, 450 g Triäthylenglykol und 134 g 1.1.1-Trimethylolpropan wurden wie in A 1 unter Katalysator-Zusatz zur Veresterung gebracht. Der erhaltene Polyester hatte eine Säurezahl von $< 2$ mg KOH/g, eine Hydroxylzahl von 175-183 mg KOH/g und eine Viskosität (bei 25 °C) von ca. 300 Pa · s.

B. Herstellung der Isocyanatkomponenten

1. Zu 1 014 Gew.T. TCDI wurden bei 80 °C innerhalb von 6 h 9,4 Gew.-T. Wasser zugegeben. Nach Beendigung der $CO_2$-Entwicklung, es entweichen ca. 12 l $CO_2$, wurde noch eine weitere Stunde bei 80 °C erhitzt. Das so hergestellte harnstoffgruppenhaltige Diisocyanat hatte einen NCO-Gehalt von 30,2 % und bei Raumtemperatur eine Viskosität von 15 Pa · s.

2. Durch 1 014 Gew.-T. TCDI wurde bei 80 °C ein mit Wasser gesättigter Stickstoffstrom hindurchgeleitet. Nach dem auf diese Weise 9,4 Gew.-T. Wasser zugesetzt waren, wurde der Stickstoffstrom beendet und noch ca. 1 h bei 80 °C weiter erhitzt. Das so hergestellte harnstoffgruppenhaltige Diisocyanat hatte einen NCO-Gehalt von 30,2 % und bei Raumtemperatur eine Viskosität von 11,5 Pa · s.

3. Zu 1 023 Gew.-T. TCDI wurden bei 80 °C analog den bei B 1. angegebenen Bedingungen 14.7 Gew.-T. Wasser zugegeben. Nach Beendigung der $CO_2$-Entwicklung (ca. 18 l) hatte das Reaktionsprodukt einen NCO-Gehalt von 28 % und bei Raumtemperatur eine Viskosität von 250 Pa · s.

4. Werden 1 023 Gew.-T. TCDI mit 14,7 Gew.-T. Wasser unter den unter B 2. angegebenen Bedingungen umgesetzt, so besitzt das Reaktionsprodukt einen NCO-Gehalt von 28 % und bei Raumtemperatur eine Viskosität von ca. 230 Pa · s.

C. Verwendung der isocyanatomethylgruppenhaltigen Derivate des TCDI zur Herstellung von Polyurethan-Elastomeren

Zur Herstellung der Normprüfkörper zur Ermittlung der mechanischen Kenndaten, sowie für die Stabilitätsprüfungen wurde zunächst die Komponente A, bestehend aus dem Polyester mit Zeolithpaste (50 %ige Suspension von Molekularsieb-Zeolith in Rizinusöl), Stabilisatoren, Verdünner PU (Monoalkohol der Fa. Bayer ; herstellt aus Phenol und Propylenoxid), Füllstoffen, Entschäumer und gegebenenfalls mit Weichmacher (Butylbenzylphthalat ; Hersteller : Fa. Bayer) und mit Pigmenten bei 20 °C bis 80 °C innig gemischt und anschließend bis zur Blasenfreiheit entgast.

Danach wurde die Komponente B (Isocyanatkomponente = isocyanatomethylgruppenenthaltendes Derivat des TCDI) zum Komponentengemisch A zugesezt, homogenisiert und bis zur Blasenfreiheit entgast. Die Mischungen blieben 20-40 Minuten gießfähig und erstarrten innerhalb 24 h zu einem vernetzten Polyurethan-Kunststoff. Das OH/NCO-Gruppenverhältnis betrug in allen Beispielen 1 : 1. Die folgenden Tabelle zeigt die mechanischen Kenndaten einiger Polyurethan-Elastomere :

(Siehe die Tabelle, Seiten 5 und 6)

| Rezeptur | | I | II | III | IV | V | VI | VII | VIII |
|---|---|---|---|---|---|---|---|---|---|
| | Dimension | | | | | | | | |
| Komponente A | | | | | | | | | |
| Polyester gemäß A 1 | Gew.-T. | - | - | - | - | 305,33 | - | - | 295,66 |
| Polyester gemäß A 2 | Gew.-T. | 368,42 | 424,71 | - | - | - | 361,09 | - | - |
| Polyester gemäß A 3 | Gew.-T. | - | - | 361,13 | 281,36 | - | - | 273,76 | - |
| Schiefermehl | Gew.-T. | 430 | 375 | 380 | 370 | - | 430 | 370 | - |
| Titandioxid | Gew.-T | 20 | - | 20 | - | - | 20 | - | - |
| Kaolin | Gew.-T | - | - | - | 65 | - | - | 65 | - |
| Quarzmehl | Gew.-T | - | - | - | - | 450 | - | - | 450 |
| Chromoxidgrün | Gew.-T | - | 25 | - | 20 | - | - | 20 | - |
| Chromoxidgelb | Gew.-T | - | - | - | 5 | - | - | 5 | - |
| Verdünner PU | Gew.-T | 30 | 20 | 20 | 10 | - | 30 | 10 | - |
| Zeolithpaste | Gew.-T | 30 | 30 | 30 | 50 | 30 | 30 | 50 | 30 |
| Butylbenzylphthalat | Gew.-T | - | - | - | 50 | - | - | 50 | - |
| UV-Stabilisator | Gew.-T | 2,5 | 2,5 | 2,5 | 2,5 | - | 2,5 | 2,5 | - |
| Oxidationsstabilisator | Gew.-T | 2,5 | 2,5 | 2,5 | 2,5 | - | 2,5 | 2,5 | - |
| Entschäumer | Gew.-T | 0,5 | 0,5 | 0,5 | 0,5 | 0,5 | 0,5 | 0,5 | 0,5 |
| Komponente B | | | | | | | | | |
| Isocyanat gemäß B 1 | Gew.-T | 116,08 | 119,79 | 183,37 | 143,14 | 214,17 | - | - | - |
| Isocyanat gemäß B 3 | Gew.-T | - | - | - | - | - | 123,41 | 150,74 | 223,84 |

| Rezeptur | | I | II | III | IV | V | VI | VII | VIII |
|---|---|---|---|---|---|---|---|---|---|
| Mechanische Kenndaten | Dimension | | | | | | | | |
| <u>Härte nach DIN 53 505</u> | | | | | | | | | |
| Shore A | | 42 | 53 | 79 | 81 | 99 | 45 | 87 | 98 |
| Shore D | | - | - | 30 | 34 | 75 | - | 36 | 77 |
| <u>Zugversuch nach DIN 53 504</u> | | | | | | | | | |
| Bruchspannung | $N/mm^2$ | 1,38 | 1,41 | 4,2 | 2,9 | 25,8 | 1,47 | 3,1 | 26,7 |
| Bruchdehnung | % | 420 | 320 | 150 | 115 | 20 | 400 | 100 | 18 |
| <u>Weiterreißversuch nach DIN 53 507</u> | | | | | | | | | |
| Weiterreißwiderstand | N/mm | 4,5 | 4,0 | 3,1 | - | - | 4,9 | - | - |
| <u>Weiterreißwiderstand nach DIN 53 515</u> | | | | | | | | | |
| nach Graves | N/mm | 9 | 8,1 | 5,8 | 9,4 | 56,8 | 9,6 | 9,7 | 57,2 |
| <u>Abrieb nach DIN 53 516</u> | | | | | | | | | |
| Volumenverlust | $mm^3$ | 80 | 50 | 45 | 51 | 60 | 75 | 53 | 57 |
| <u>Druckverformungsrest nach DIN 53 517</u> | | | | | | | | | |
| 70 h / 20 °C | % | 9 | 9 | 4 | 9 | - | 8 | 5 | - |

Auch beim Xenotest 450 LF (der Fa. Original Hanau) bleiben die Probekörper nach einer Prüfdauer von 2 000 Std. nahezu unverändert. Die Shore-Härten und Zugfestigkeiten waren geringfügig gesunken, wobei die Dehnung wenig angestiegen war. Weiterreißwiderstand, Abrieb und Druckverformungsrest blieben nahezu unverändert.

Die auf diese Weise hergestellten lösungsmittelfreien Polyurethan-Elastomere lassen sich vorzüglich als Bodenbeläge für Industrie- und Sportanlagen, für Schulhöfe und dergleichen verwenden. Auch als Kleber, Kitte, Dichtungs- und Fugenvergußmassen sind sie geeignet. Auch für die Herstellung von Druckwalzen haben sie sich bewährt. Ein weiteres Anwendungsgebiet ist die Beschichtung von Substraten und Geweben aller Art, z. B. für die Herstellung von Transportbändern, Teppichrücken und Matten. Ferner lassen sich Bodenfliesen und Schaumversiegelungen damit herstellen.

Entstehen bei der Hydrolyse des TDCI feste Harnstoffe, ist die Verwendung von Lösungsmitteln für die Reaktion von Vorteil. Geeignete PUR-Lösungsmittel sind solche, die inert gegen NCO-Gruppen sind, wie beispielsweise Aromaten, Hydroaromaten und chlorierte Aromaten, wie Benzol, Toluol, Diisopropropylbenzol, Chlorbenzol und o-Dichlorbenzol u. a.

## Ansprüche

1. Isocyanatomethylgruppenhaltige Harnstoffderivate des 3(4), 8(9)-Diisocyanatomethyltricyclo [5.2.1.0$^{2,6}$] decans der Formel

$$OCN—H_2C—[R—CH_2—NH—CO—NH—CH_2]_n—R—CH_2—NCO,$$

in der R

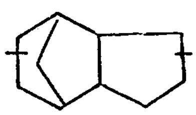

und n = 1-5 ist.

2. Verfahren zur Herstellung der isocyanatomethylgruppenhaltigen Harnstoffderivate gemäß Anspruch 1, dadurch gekennzeichnet, daß man 3(4), 8(9)-Diisocyanatomethyltricyclo [5.2.1.0$^{2,6}$] decan mit Wasser im molaren Verhältnis von 12-2,0 : 1 bei Temperaturen von 60-110 °C, gegebenenfalls in Gegenwart von Katalysatoren, umsetzt.

3. Verfahren nach Anspruch 2, dadurch gekennzeichnet, daß man das Diisocyanat mit dem Wasser im Molverhältnis von 6-3 : 1 umsetzt.

4. Verfahren nach Anspruch 3, dadurch gekennzeichnet, daß man die Umsetzung bei Temperaturen von 80-100 °C durchführt.

5. Verfahren zur Herstellung der isocyanatomethylgruppenhaltigen Harnstoffderivate nach den Ansprüchen 2-4, dadurch gekennzeichnet, daß man einen mit Wasser gesättigten Stickstoffstrom so lange auf den genannten Temperaturbereich aufgewärmtes Diisocyanat hindurchleitet, bis 1 Teil Wasser auf 12 bis 2,0 Teile Diisocyanat zugeführt worden sind.

6. Verwendung der isocyanatomethylgruppenhaltigen Harnstoffderivate nach Anspruch 1 zur Herstellung von lösungsmittelfreien Polyurethan-Elastomeren.

## Claims

1. Urea derivates containing isocyanatomethyl groups of 3(4), 8(9)-diisocyanatomethyltricyclo 5.2.1.0$^{2,6}$ decane of the formula

$$OCN—H_2C—[R—CH_2—NH—CO—NH—CH_2]_n—R—CH_2—NCO,$$

in which R is

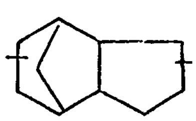

and n = 1-5.

2. Process for the production of urea derivates containing isocyanatomethyl groups according to Claim 1, wherein 3(4), 8(9)-diisocyanatomethyltricyclo [5.2.1.0$^{2,6}$] decane is mixed with water in a molar

ratio of 12-2.0 : 1 at temperature of 60-110 °C, and possibly in the presence of catalysts.

3. Process according to Claim 2, wherein the diisocyanate is mixed with water in a molar ratio of 6-3 : 1.

4. Process according to Claim 3, wherein the mixing is carried out at temperatures of 80-100 °C.

5. Process for producing urea derivates containing isocyanatomethyl groups according to Claims 2 to 4, wherein a stream of water saturated nitrogen is passed through the diisocyanate, warmed to the temperature mentioned above, until 1 part of water has been supplied to 12 to 20 parts of diisocyanate.

6. Use of the urea derivates containing isocyanatomethyl groups according to Claim 1 to produce polyurethane elastomers free of solvents.

**Revendications**

1. Dérivés uréiques de 3(4), 8(9)-diisocyanatométhyltricyclo (5.2.1.$0^{2,6}$) décan (TCDI) répondant à la formule

$$OCN—H_2C—(R—CH_2—NH—CO—NH—CH_2)_n—R—CH_2—NCO$$

où R est

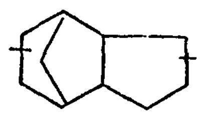

et n – 1 à 5
et n = 1 à 5
que l'on peut obtenir facilement ces composés en faisant réagir du 3(4), 8(9)-diisocyanatométhyltricyclo (5.2.1.$0^{2,6}$) décane (TCDI)

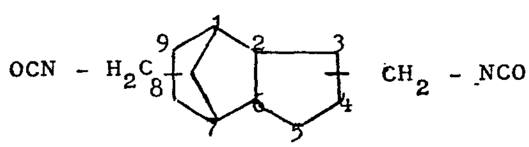

2. Procédé pour la fabrication de dérivés uréiques contenant des groupes isocyanatométhyle suivant la revendication 1, caractérisé en ce que l'on fait réagir du 3(4), 8(9)-diisocyanatométhyltricyclo (5.2.1. $0^{2,6}$) décane avec de l'eau, dans le rapport molaire de 12 à 2 : 1, à des températures de 60 à 110 °C, éventuellement en présence de catalyseurs.

3. Procédé suivant la revendication 2, caractérisé en ce que l'on fait réagir le diisocyanate avec de l'eau, dans le rapport molaire de 6 à 3 à 1.

4. Procédé suivant la revendication 3, caractérisé en ce que l'on effectue la réaction à des températures de 80 à 100 °C.

5. Procédé pour la fabrication de dérivés uréiques contenant des groupes isocyanatométhyle suivant les revendications 2 à 4, caractérisé en ce que l'on fait passer un courant d'azote saturé d'eau sur le diisocyanate chauffé à une température de l'ordre mentionné, assez longtemps pour qu'il soit fourni une partie en poids d'eau pour 12 à 2 parties en poids de diisocyanate.

6. Utilisation des dérivés uréiques contenant des groupes isocyanatométhyle suivant la revendication 1, pour la fabrication de polyuréthanes élastomères exempts de solvants.